# EUROPÄISCHE PATENTSCHRIFT

(11) **EP 0 672 398 B1**
(45) Veröffentlichungstag und Bekanntmachung des Hinweises auf die Patenterteilung: **07.04.1999**
(21) Anmeldenummer: 95102056.9
(22) Anmeldetag: 15.02.1995
(51) Int. Cl.: A61F 2/64

(54) **Schwenkverbindung zwischen Teilen eines orthopädischen Hilfsmittels**
Swivelling connection between parts of an orthopaedic device
Liaison pivotante entre des parties d'un dispositif orthopédique

(30) Priorität: 22.02.1994 NL 9400269
(43) Veröffentlichungstag der Anmeldung: 20.09.1995
(73) Patentinhaber: Otto Bock Orthopädische Industrie Besitz- und Verwaltungs-Kommanditgesellschaft, 37115 Duderstadt (DE)
(72) Erfinder: Van de Veen, P.G., 7514 EC Enschede (NL)
(74) Vertreter: Gramm, Werner, Prof. Dipl.-Ing.

(56) Entgegenhaltungen:
- EP-A- 0 482 809
- WO-A-92/22267
- DE-C- 414 982
- GB-A- 1 303 738
- GB-A- 2 004 753

## Beschreibung

Die Erfindung betrifft eine Schwenkverbindung zwischen Teilen eines orthopädischen Hilfsmittels, insbesondere in der Art eines Kniegelenkes einer Prothese für Beinamputierte, bestehend aus einer mehrgliedrigen kinematischen Gelenkkette mit mindestens vier Gelenkgliedern, in der die jeweils miteinander verbundenen Glieder eine gemeinsame Drehachse aufweisen, und diese Drehachsen im wesentlichen parallel zueinander verlaufen, wobei ein streckseitig angeordnetes Gelenkglied nur mit einem oberen Anschluß-Gelenkglied und einem unteren Anschluß-Gelenkglied drehbar verbunden ist, und ein beugeseitig angeordnetes Gelenkglied mit seinem oberen Ende an dem oberen Anschluß-Gelenkglied drehbar und mit seinem unteren Ende über eine zwischengeschaltete Gelenkverbindung an dem unteren Anschluß-Gelenkglied drehbar angelenkt ist.

Die Verwendung mehrgliedriger Gelenksysteme in orthopädischen Hilfsmitteln zum Ersetzen oder Unterstützen der natürlichen Gelenkfunktion ist seit langem bekannt und erfolgt häufig wegen der Vorteile, die solche Systeme gegenüber herkömmlicheren Gelenkmechanismen mit einem einzigen, ortsfesten Drehzentrum aufweisen. Diese Vorteile umfassen u. a. ein besseres Nachfolgen oder eine bessere Nachahmung der Bewegung der natürlichen Gliedmaßen im Vergleich zu einachsigen Gelenkmechanismen sowie eine erhöhte und besser kontrollierbare Gelenkstabilität unter Belastung und werden durch eine geeignete Wahl der Abmessungen der Glieder des Gelenksystems und ihrer Stellung zueinander erreicht. Letzteres ist besonders dann von großer Bedeutung, wenn ein mehrgliedriges Gelenksystem zum Ersetzen oder Unterstützen der natürlichen Kniefunktion verwendet wird. Zu Beginn eines Schrittes, wenn die Ferse den Boden berührt und der Gelenkmechanismus in gestreckter Stellung durch das Gewicht des Nutzers belastet wird, ist es wichtig, daß der Mechanismus nicht sofort beugt, weil der Nutzer dann keinerlei Körperunterstützung hätte und fallen würde. In einem einachsigen Gelenkmechanismus kann dies nur durch den Einsatz eines komplizierten und nicht sehr zuverlässigen Bremsmechanismus verhindert werden. In einem mehrgliedrigen Gelenksystem kann diese Eigenschaft hingegen durch Wahl der Gelenkgeometrie dergestalt erreicht werden, daß das virtuelle Drehzentrum des Mechanismus, um das der Unterschenkel der Beinprothese oder -orthese schwenkt, hinter der Wirkungslinie zwischen den beiden Belastungspunkten (Ferse und Hüftgelenk) liegt.

Mit einer geeigneten Wahl der Geometrie des mehrgliedrigen Gelenksystems kann darüberhinaus erreicht werden, daß die Lage des virtuellen Drehzentrums am Ende des Schrittes eine im Vergleich zu einachsigen Gelenkmechanismen leichtere Einleitung des Vorschwingens der Beinprothese oder -orthese erlaubt. Die große Stabilität zu Beginn des Schrittes, die leichte Einleitung des Vorschwingens sowie die Möglichkeit, auf einfache Weise einen guten Kompromiß zwischen diesen beiden Merkmalen zu finden, verleihen dem mehrgliedrigen Gelenksystem eine hervorragende Eignung zum Ersetzen oder Unterstützen einer natürlichen Gelenkfunktion.

Eine wichtige Anforderung an ein Gelenksystem in Gestalt einer mehrgliedrigen kinematischen Gelenkkette in solchen Anwendungen besteht darin, daß es zu Beginn einer Belastung, wie das natürliche Knie, eine Beugung ausführt. Der Dämpfungseinfluß des Gelenkes bei Belastung des Beines verhindert einen ruckartigen Verlauf der Beugebewegung, der für den Nutzer unangenehm und langfristig sogar schmerzhaft sein kann. Die Anfangsbeugung unter Belastung verhindert ferner eine Begrenzung der Vertikalbewegung des Körperschwerpunktes, wodurch die zum Gehen erforderliche Energie sich in Grenzen hält. Diese Art der Kniebeugung wird im Englischen mit "Stance Flexion" bezeichnet, was soviel wie "Standphasen-Beugung" bedeutet.

Die bestehenden Schwenkverbindungen zwischen Teilen orthopädischer Hilfsmittel, insbesondere in der Art eines Kniegelenkes einer Prothese für Beinamputierte, haben den Nachteil, daß die "Standphasen-Beugung" auf 3 bis 4 Grad begrenzt ist. Dies reicht zur vollständigen Nutzung der genannten Vorteile der "Standphasen-Beugung" nicht aus. Ein weiterer Nachteil entsteht aus dem weit zur Beugeseite verlagerten Anschlußpunkt der Prothese. Insbesondere im Fall der sogenannten "Knie-Exartikulation" (einer Abtrennung des Unterschenkels im Kniespalt bei vollständigem Erhalt des zum Oberschenkel gehörenden Teils des Kniegelenkes) erreicht diese Verlagerung unannehmbare Werte.

Die eingangs beschriebene Schwenkverbindung läßt sich der EP-A-0 482 809 entnehmen. Hier besteht die das untere Ende des beugeseitigen Gelenkgliedes mit dem unteren Anschluß-Gelenkglied verbindende zwischengeschaltete Gelenkverbindung nur aus einem Koppelglied, das mit seinem oberen Ende am beugeseitigen Gelenkglied und mit seinem unteren Ende am unteren Anschluß-Gelenkglied angelenkt ist. Es handelt sich somit um eine fünfgliedrige kinematische Gelenkkette mit zwei Freiheitsgraden, so daß diese Gelenkkette für ein Kniegelenk unbrauchbar wäre. Vorgesehen ist daher zusätzlich noch ein Federglied, das mit seinem einen Ende an dem oberen Anschluß-Gelenkglied und seinem unteren Ende an einer Nase des beugeseitigen Gelenkgliedes angelenkt ist. Dieses Federglied übt auf die Nase des beugeseitigen Gelenkgliedes ständig einen Druck nach unten aus, drückt dadurch das beugeseitige Gelenkglied gegen einen Anschlag und blockiert somit das Kniegelenk in seiner Streckstellung. Eine Kniebeugung wird somit verhindert und erst dadurch ermöglicht, daß über ein an der genannten Nase des beugeseitigen Gelenkgliedes angreifendes Zugkabel eine manuell aufzubringende, nach oben gerichtete Zugkraft ausgeübt wird. Eine Aufhebung der Zugkraft führt zur sofortigen Blockierung des Kniegelenkes in der jeweils gerade erreichten Beugestellung. Diese Ausführungsform läßt somit keine freie Schwenkbewegung zu. Ausgehend von der Streckstellung des Kniegelenkes ist - beim Aufbringen der genannten Zugkraft - eine Verschwenkung des beugeseitigen Gelenkgliedes lediglich in von dem streckseitigen Gelenkglied wegweisenden Richtung möglich. Eine "Standphasen-Beugung" ist somit ausgeschlossen.

Ziel der Erfindung ist es, eine Schwenkverbindung des in der Einleitung angegebenen Typs zur Verfügung zu stellen, die eine einfache Konstruktion aufweist, zuverlässig im Gebrauch ist und eine Standphasen-Beugung zuläßt.

Ausgehend von der eingangs beschriebenen Schwenkverbindung wird diese Aufgabe erfindungsgemäß dadurch gelöst, daß die zwischengeschaltete Gelenkverbindung zwei Drehverbindungen am beugeseitigen Gelenkglied und zwei Drehverbindungen am unteren Anschluß-Gelenkglied aufweist, so daß bei Bewegung der Schwenkverbindung das beugeseitig angeordnete Gelenkglied gegenüber dem unteren Anschluß-Gelenkglied eine zwangsläufige Bewegung ausführt, die sowohl Translation als auch Rotation umfaßt.

Die erfindungsgemäße Schwenkverbindung liefert eine gewünschte Funktionalität und eine relativ große "Standphasen-Beugung" bei gleichzeitiger Verhinderung eines zweiten Freiheitsgrades. Die "Standphasen-Beugung" ist groß genug, um ein natürliches Gehen zu ermöglichen, in der die eine Schwenkverbindung ("Standphasen-Beugung") die andere Schwenkverbindung ("Schwungphasen-Beugung") blockiert. Die erfindungsgemäße Schwenkverbindung ermöglicht es, den Anschlußpunkt für ein orthopädisches Hilfsmittel ausreichend weit zur Streckseite zu legen und zwar auch im Fall einer Knie-Exartikulation.

Eine bevorzugte Ausführungsform der erfindungsgemäßen Schwenkverbindung ist dadurch gekennzeichnet, daß die zwischengeschaltete Gelenkverbindung unter anderem ein Koppelglied aufweist, das sowohl an dem beugeseitigen Gelenkglied als auch am unteren Anschluß-Gelenkglied schwenkbar angelenkt ist. - Eine weitere bevorzugte Ausführungsform der erfindungsgemäßen Schwenkverbindung ist dadurch gekennzeichnet, daß die zwischengeschaltete Gelenkverbindung zwei Koppelglieder aufweist, die jeweils sowohl an dem beugeseitigen Gelenkglied als auch am unteren Anschluß-Gelenkglied schwenkbar angelenkt sind und die Glieder einer sogenannten Stephensonschen Kette (sechsgliedrige zwangsläufige Gelenkkette nach Stephenson) bilden. - In der einfachen Ausführung mit zwei Koppelgliedern führt das beugeseitige Koppelglied gegenüber dem unteren Anschluß-Gelenkglied eine zwangsläufige Bewegung mit translatorischen und rotatorischen Komponenten aus, ohne daß es zusätzlicher technischer Maßnahmen bedarf. In der Ausführung mit einem Koppelglied ist zum Erzielen einer zwangsläufigen Bewegung mit translatorischen und rotatorischen Komponenten eine zusätzliche technische Maßnahme (getriebetechnische Ergänzung) nötig.

Eine weitere bevorzugte Ausführungsform der erfindungsgemäßen Schwenkverbindung ist dadurch gekennzeichnet, daß die zwischengeschaltete Gelenkverbindung unter anderem eine dreigliedrige Gelenkkette aufweist, die aus einem mit dem beugeseitigen Gelenkglied drehbar verbundenen Glied besteht, das seinerseits an zwei verschiedenen Stellen mit den beiden Verbindungsgliedern drehbar verbunden ist, die ihrerseits beide mit dem unteren Anschluß-Gelenkglied drehbar verbunden sind. - Eine noch speziellere Ausführung der erfindungsgemäßen Schwenkverbindung ist dadurch gekennzeichnet, daß das beugeseitige Gelenkglied durch Zwischenschaltung zweier dreigliedriger Gelenkketten mit dem unteren Anschluß-Gelenkglied verbunden ist. - Hierbei handelt es sich um Varianten der erfindungsgemäßen Schwenkverbindung gemäß den Ansprüchen 2 und 3, bei denen die Koppelglieder durch die dreigliedrigen Gelenkketten ersetzt worden sind.

Eine weitere bevorzugte Ausführungsform der erfindungsgemäßen Schwenkverbindung ist dadurch gekennzeichnet, daß die zwischengeschaltete Gelenkverbindung unter anderem ein aus Bolzen und Gleitführung gebildetes Schubgelenk aufweist. Eine spezielle Gestaltungs-Variante dieser Ausführung entsteht dadurch, daß das beugeseitige Gelenkglied mittels zweier Schubgelenke mit dem unteren Anschluß-Gelenkglied verbunden wird. In diesen Ausführungen ist ein Koppelglied wie zum Beispiel gemäß den Ansprüchen 2 und 3 oder eine dreigliedrige Gelenkkette wie in den Ansprüchen 4 und 5 angegeben durch ein aus Bolzen und Gleitführung gebildetes Schubgelenk ersetzt worden. Auch mit diesen technischen Maßnahmen führt das beugeseitige Gelenkglied gegenüber dem unteren Anschluß-Gelenkglied eine zwangsläufige Bewegung mit translatorischen und rotatorischen Komponenten aus.

Eine bevorzugte Ausführungsform der erfindungsgemäßen Schwenkverbindung ist dadurch gekennzeichnet, daß zumindest eines der Glieder paarweise angeordnet ist. Diese mehrfach angeordneten Glieder erhöhen die Stabilität der Schwenkverbindung.

Die Schwenkverbindung enthält vorzugsweise zumindest ein federndes Element, das mit zumindest zwei der Glieder verbunden ist. Eine bevorzugte Ausführung dieses federnden Elementes enthält einen Dämpfer. Die Feder kann zum Beispiel zur Erleichterung des Vorschwingens des Unterschenkels am Ende der Schwungphase des Gehens genutzt werden. Der Dämpfer kann zum Beispiel zur Energieumwandlung und -ableitung während der Schwingbewegung genutzt werden.

Weitere Merkmale der vorliegenden Erfindung werden anhand der in den nachfolgenden Figuren dargestellten Ausführungsbeispiele erläutert. Es zeigen:
- Figur 1: eine Seitenansicht einer Prothese für Beinamputierte;
- Figur 2: eine Perspektive eines teilweise geschnittenen Teilbereiches der in Figur 1 dargestellten Prothese;
- Figuren 3-9: schematische Ansichten eines erfindungsgemäßen sechsgliedrigen Gelenksystems;
- Figur 10: eine schematische Darstellung eines alternativen Gelenksystems, ausgestattet mit zwei aus Bolzen und Gleitführungen gebildeten Schubgelenken und
- Figur 11: eine schematische Darstellung eines weiteren alternativen Gelenksystems, ausgestattet mit 10 Gliedern.

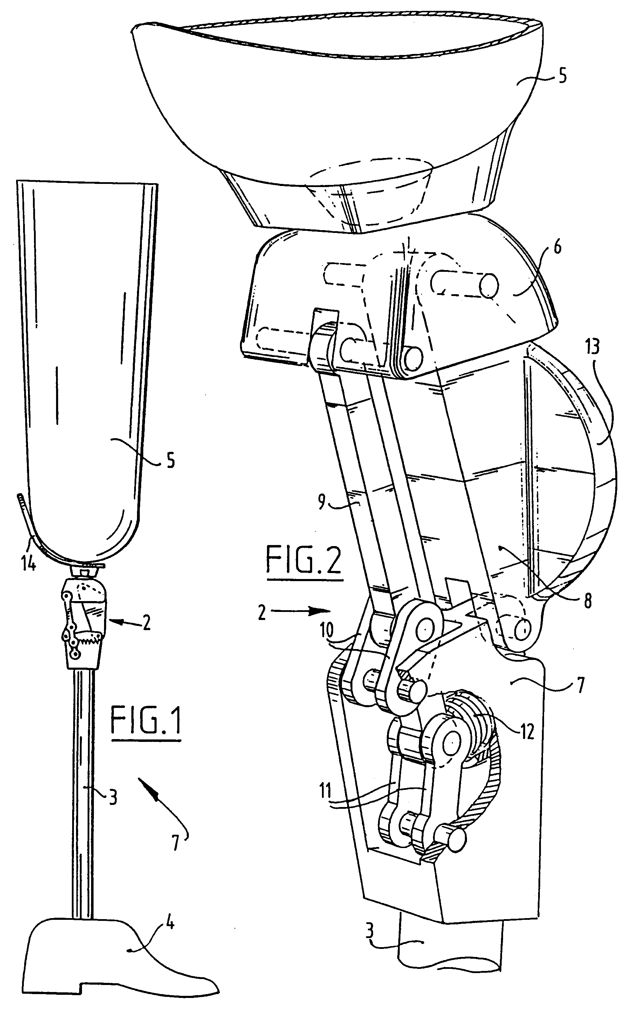

Figur 1 zeigt eine Prothese für Beinamputierte, in der ein Gelenksystem 2 die Funktion des Kniegelenkes erfüllt. Die Prothese 1 enthält ferner einen künstlichen Unterschenkel 3 mit künstlichem Fuß 4 sowie ein Mittel 5 zur Befestigung der Prothese 1 am Amputationsstumpf.

Figur 2 zeigt das Gelenksystem 2 im einzelnen. Das in dieser Figur dargestellte sechsgliedrige Gelenksystem 2 setzt sich zusammen aus einem oberen Anschluß-Gelenkglied 6, das mit dem Mittel 5 zur Befestigung der Prothese 1 am Aputationsstumpf verbunden ist, einem unteren Anschluß-Gelenkglied 7, das mit dem künstlichen Unterschenkel 3 verbunden ist, einem streckseitig angeordneten, nur mit den Anschluß-Gelenkgliedern 6, 7 drehbar verbundenen Gelenkglied 8, einem beugeseitig angeordneten, mit dem oberen Anschluß-Gelenkglied 6 drehbar verbundenen Gelenkglied 9 sowie zwei Koppelgliedern 10, 11, die beide sowohl mit dem beugeseitigen Gelenkglied 9 als auch mit dem unteren Anschluß-Gelenkglied 7 drehbar verbunden sind. Zur Erhöhung der Stabilität des Gelenksystems 2 sind die Koppelglieder 10, 11 paarweise angeordnet. Die Figur zeigt ebenfalls ein federndes Element 12 zur Erleichterung des Vorschwingens des künstlichen Unterschenkels 3. Das streckseitige Gelenkglied 8 ist mit einem kniescheibenförmigen Bereich versehen, um seinem Äußeren das Erscheinungsbild eines menschlichen Knies zu verleihen. Die Arbeitsweise dieses Gelenksystems 2 wird anhand der nachfolgenden Figuren naher erläutert.

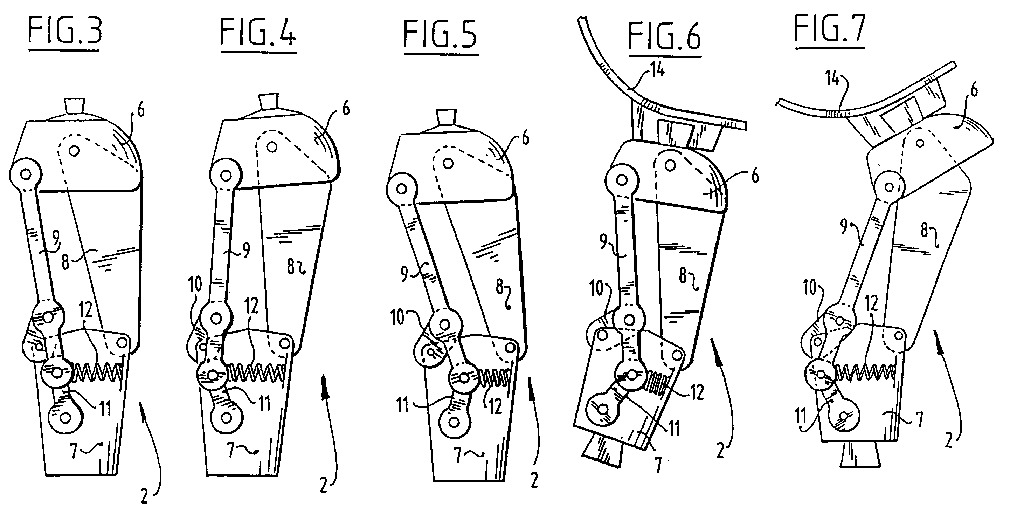

Figuren 3 - 5 zeigen schematisch das Gelenksystem 2 der Figur 2 in Seitenansicht. Die in Figur 3 dargestellte Stellung stellt die Ausgangsstellung dar. In dieser Stellung ist das Gelenksystem 2 nicht belastet (der Amputationsstumpf stützt sich nicht auf der Prothese 1 ab), und Prothese 1 liegt auf einer Linie mit dem Amputationsstumpf (das "Bein" ist gestreckt). Das untere Ende des beugeseitigen Gelenkgliedes 9 befindet sich in Ausgangsstellung auf dem höchsten Punkt seiner Bewegungsbahn. Figur 4 zeigt das Gelenksystem 2 in einer Ausgangsstellung für die normale Kniebeugung, der sogenannten Schwungphasen-Beugung. Das untere Ende des beugeseitigen Gelenkgliedes 9 hat sich gegenüber der Stellung in Figur 3 zur Beugeseite hin nach unten verlagert. Diese Verlagerung erfolgt gegen die Wirkung des federnden Elementes 12, wodurch auf das Gelenksystem 2 eine Kraft ausgeübt wird, die es in seine Ausgangsstellung zurückführen wird, wenn keine äußeren Kräfte auf das System einwirken. Figur 5 zeigt das Gelenksystem 2 zu Beginn der Standphase des Gehens in einer Ausgangsstellung für die sogenannte Standphasen-Beugung, die beim Fersenkontakt beginnt und sich während der Standphase fortsetzt. Diese Beugebewegung sorgt für Stoßabsorption und ein natürliches Gangbild. Diese Figur zeigt den Beginn der "Standphasen-Beugung". Das untere Ende des beugeseitigen Gelenkgliedes 9 hat sich gegenüber der Stellung in Figur 3 zur Streckseite hin nach unten verlagert.

Figur 6 zeigt das Gelenksystem 2 in einer gegenüber der Darstellung in Figur 5 fortgeschrittenen "Standphasen-Beugung". Ferner sichtbar ist ein Verbindungsteil 14, wodurch das Gelenksystem 2 besonders für die prothetische Versorgung von Patienten mit einer Knie-Exartikulation geeignet ist. Eine Knie-Exartikulation ist eine Amputation, die wortgetreu im Kniespalt trennt.

Figur 7 zeigt das Gelenksystem 2 mit Verbindungsteil 14 in einer gegenüber der Darstellung in Figur 4 fortgeschrittenen "Schungphasen-Beugung".

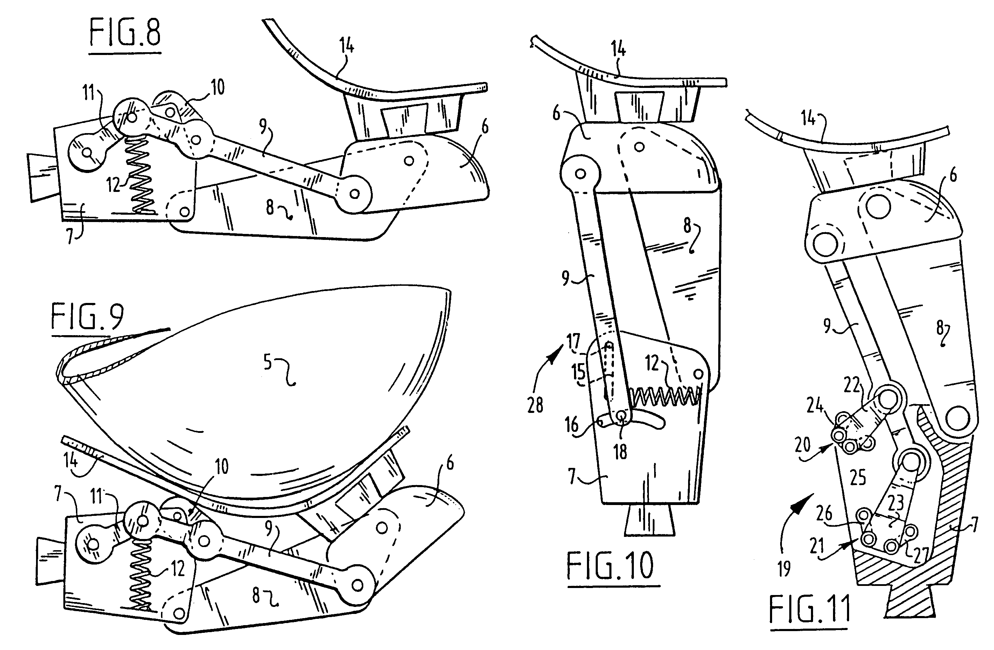

Figur 8 zeigt dasselbe Gelenksystem 2 in einer gegenüber der Darstellung in Figur 7 noch weiter fortgeschrittenen "Schwungphasen-Beugung".

Figur 9 zeigt das Gelenksystem 2 in der maximalen Beugestellung.

In der weit fortgeschrittenen "swing flexion", wie in den Figuren 8 und 9 gezeigt, ist deutlich zu erkennen, daß das streckseitige Gelenkglied 8 wie eine "Kniescheibe" wirkt. Das Gelenksystem 2 ist so ausgeführt, daß es sogar möglich ist, sich am streckseitigen Gelenkglied 8 abzustützen, ohne daR das Gelenksystem 2 dadurch schädlichen Wirkungen ausgesetzt wird. Eine solche Abstützung tritt zum Beispiel beim Knien auf.

Figur 10 zeigt ein alternatives Gelenksystem 28 in der Ausgangsstellung. Das untere Anschluß-Gelenkglied 7 ist mit Gleitführungen 15, 16 ausgestattet, die mit den am unteren Ende des beugeseitigen Gelenkgliedes 9 befestigten Bolzen 17, 18 ein Schubgelenk bilden. Bei einer Schwenkbewegung des Gelenksystems 28 wird das beugeseitige Gelenkglied 9 gegenüber dem unteren Anschluß-Gelenkglied 7 wegen der Führung der Bolzen 17, 18 in den Gleitbahnen 15, 16 eine zwangsläufige Bewegung ausführen.

Figur 11 zeigt ein anderes Ausführungsbeispiel der erfindungsgemäßen Schwenkverbindung. Bei diesem Mechanismus ist das beugeseitige Gelenkglied 9 durch Zwischenschaltung zweier dreigliedriger Gelenkketten 20, 21 mit dem unteren Anschluß-Gelenkglied 7 verbunden. Diese dreigliedrigen Gelenkketten 20, 21 bestehen aus einem mit dem beugeseitigen Gelenkglied 9 drehbar verbundenen Glied 22, 23 und zwei Verbindungsgliedern 24, 25, 26, 27, die mit dem Glied 22, 23 verbunden und mit dem unteren Anschluß-Gelenkglied 7 drehbar verbunden sind.

Es dürfte einleuchtend sein, daß es ebenfalls möglich ist, Elemente der in den vorstehenden Figuren dargestellten Gelenksysteme 2, 28, 19 miteinander zu kombinieren. So kann zum Beispiel ein Koppelglied 10, 11 mit einem Schubglied bestehend aus Gleitführung 15, 16 und Bolzen 17, 18 oder einer dreigliedrigen Gelenkkette 20, 21 kombiniert werden. Ein Beispiel für eine andere Kombination besteht aus einem Gelenksystem 2, 28, 19 mit einem Schubgelenk bestehend aus einer Gleitführung 15, 16 und Bolzen 17, 18 in Verbindung mit einer dreigliedrigen Gelenkkette 20, 21.

## Patentansprüche

1. Schwenkverbindung zwischen Teilen eines orthopädischen Hilfsmittels, insbesondere in der Art eines Kniegelenkes einer Prothese für Beinamputierte, bestehend aus einer mehrgliedrigen kinematischen Gelenkkette mit mindestens vier Gelenkgliedern (6, 7, 8, 9), in der die jeweils miteinander verbundenen Glieder eine gemeinsame Drehachse aufweisen, und diese Drehachsen im wesentlichen parallel zueinander verlaufen, wobei ein streckseitig angeordnetes Gelenkglied (8) nur mit einem oberen Anschluß-Gelenkglied (6) und einem unteren Anschluß-Gelenkglied (7) drehbar verbunden ist, und ein beugeseitig angeordnetes Gelenkglied (9) mit seinem oberen Ende an dem oberen Anschluß-Gelenkglied (6) drehbar und mit seinem unteren Ende über eine zwischengeschaltete Gelenkverbindung an dem unteren Anschluß-Gelenkglied (7) drehbar angelenkt ist, **dadurch gekennzeichnet**, daß die zwischengeschaltete Gelenkverbindung zwei Drehverbindungen am beugeseitigen Gelenkglied (9) und zwei Drehverbindungen am unteren Anschluß-Gelenkglied (7) aufweist, so daß bei Bewegung der Schwenkverbindung das beugeseitig angeordnete Gelenkglied (9) gegenüber dem unteren Anschluß-Gelenkglied (7) eine zwangsläufige Bewegung ausführt, die sowohl Translation als auch Rotation umfaßt.

2. Schwenkverbindung nach Anspruch 1, **dadurch gekennzeichnet**, daß die zwischengeschaltete Gelenkverbindung unter anderem ein Koppelglied (10, 11) aufweist, das sowohl an dem beugeseitigen Gelenkglied (9) als auch am unteren Anschluß-Gelenkglied (7) schwenkbar angelenkt ist.

3. Schwenkverbindung nach Anspruch 1 oder 2, **dadurch gekennzeichnet**, daß die zwischengeschaltete Gelenkverbindung zwei Koppelglieder (10, 11) aufweist, die jeweils sowohl an dem beugeseitigen Gelenkglied (9) als auch am unteren Anschluß-Gelenkglied (7) schwenkbar angelenkt sind und die Glieder (6, 7, 8, 9, 10, 11) einer sechsgliedrigen zwangsläufigen Gelenkkette nach Stephenson bilden.

4. Schwenkverbindung nach Anspruch 1 oder 2, **dadurch gekennzeichnet**, daß die zwischengeschaltete Gelenkverbindung unter anderem eine dreigliedrige Gelenkkette (20, 21) aufweist, die aus einem mit dem beugeseitigen Gelenkglied (9) drehbar verbundenen Glied (22, 23) besteht, das seinerseits an zwei verschiedenen Stellen mit den beiden Verbindungsgliedern (24, 25, 26, 27) drehbar verbunden ist, die ihrerseits beide mit dem unteren Anschluß-Gelenkglied (7) drehbar verbunden sind.

5. Schwenkverbindung nach Anspruch 4, **dadurch gekennzeichnet**, daß das beugeseitige Gelenkglied (9) durch Zwischenschaltung zweier dreigliedriger Gelenkketten (20, 21) mit dem unteren Anschluß-Gelenkglied (7) verbunden ist.

6. Schwenkverbindung nach Anspruch 1, 2 oder 4, **dadurch gekennzeichnet**, daß die zwischengeschaltete Gelenkverbindung unter anderem ein aus Bolzen und Gleitführung gebildetes Schubgelenk (15, 17; 16, 18) aufweist.

7. Schwenkverbindung nach Anspruch 6, **dadurch gekennzeichnet**, daß das beugeseitige Gelenkglied (9) mittels zweier aus Bolzen und Gleitführungen gebildeten Schubgelenke (15, 17; 16, 18) mit dem unteren Anschluß-Gelenkglied (7) verbunden ist.

8. Schwenkverbindung nach einem der Ansprüche 1 bis 7, **dadurch gekennzeichnet**, daß zumindest eines der Glieder (10, 11) paarweise angeordnet ist. (Figur 2)

9. Schwenkverbindung nach einem der vorgenannten Ansprüche, **dadurch gekennzeichnet**, daß zumindest ein federndes Element (12) enthalten ist, das mit zumindest zwei der Glieder (6, 7, 8, 9, 10, 11) verbunden ist.

10. Schwenkverbindung nach Anspruch 9, **dadurch gekennzeichnet**, daß das federnde Element (12) einen Dämpfer enthält.

## Claims

1. Swivelling connection between parts of an orthopaedic aid, particularly in the manner of a knee joint of a prosthesis for leg amputees, comprising a multi-membered kinematic link chain with at least four link members (6, 7, 8, 9), in which the members connected together in each case have a common axis of rotation, and these axes of rotation run substantially parallel to each other, wherein a link member (8) arranged on the extensor side is rotatably connected only to an upper connection link member (6) and a lower connection link member (7), and the upper end of a link member (9) arranged on the flexor side is rotatably linked to the upper connection link member (6) and its lower end is rotatably linked to the lower connection link member (7) via an interposed articulation, characterized in that the interposed articulation has two rotary connections on the link member (9) on the flexor side and two rotary connections on the lower connection link member (7) so that when the swivelling connection moves, the link member (9) arranged on the flexor side performs an automatic movement, which comprises both translation and rotation, with respect to the lower connection link member (7).

2. Swivelling connection according to Claim 1, characterized in that the interposed articulation has inter alia a coupling member (10, 11) which is swivel-linked both to the link member (9) on the flexor side and to the lower connection link member (7).

3. Swivelling connection according to Claim 1 or 2, characterized in that the interposed articulation has two coupling members (10, 11) each of which is swivel-linked both to the link member (9) on the flexor side and to the lower connection link member (7) and form the members (6, 7, 8, 9, 10, 11) of a six-membered, automatic link chain after Stephenson.

4. Swivelling connection according to Claim 1 or 2, characterized in that the interposed articulation inter alia has a three-membered link chain (20, 21) which consists of a member (22, 23) which is rotatably connected to the link member (9) on the flexor side and for its part is rotatably connected to the two connection members (24, 25, 26, 27) in two different places, both of which connection members are for their part rotatably connected to the lower connection link member (7).

5. Swivelling connection according to Claim 4, characterized in that the link member (9) on the flexor side is connected to the lower connection link member (7) by interposition of two three-membered link chains (20, 21).

6. Swivelling connection according to Claim 1, 2 or 4, characterized in that the interposed articulation inter alia has a prismatic joint formed of bolt and slideway (15, 17; 16, 18).

7. Swivelling connection according to Claim 6, characterized in that the link member (9) on the flexor side is connected to the lower connection link member (7) by means of two prismatic joints formed of bolts and slideways (15, 17; 16, 18).

8. Swivelling connection according to one of Claims 1 to 7, characterized in that at least one of the members (10, 11) is arranged pair-wise (Fig. 2).

9. Swivelling connection according to one of the preceding Claims, characterized in that at least one spring element (12) which is connected to at least two of the members (6, 7, 8, 9, 10, 11) is contained.

10. Swivelling connection according to Claim 9, characterized in that the spring element (12) contains a damper.

## Revendications

1. Liaison pivotante entre des parties d'un accessoire orthopédique présentant en particulier la forme d'une articulation de genou d'une prothèse pour amputé de la jambe, constituée d'une chaîne cinématique articulée à plusieurs éléments comportant au moins quatre éléments articulés (6, 7, 8, 9), dans laquelle les éléments reliés les uns aux autres présentent chacun un axe de rotation commun, et ces axes de rotation s'étendent essentiellement parallèlement l'un à l'autre, tandis qu'un élément d'articulation (8) disposé du côté extension n'est relié à rotation qu'à un élément articulé supérieur de raccordement (6) et à un élément articulé inférieur de raccordement (7), et un élément articulé (9) disposé du côté flexion est relié à rotation à son extrémité supérieure à l'élément articulé supérieur de raccordement (6) et à son extrémité inférieure à l'élément articulé inférieur de raccordement (7) par l'intermédiaire d'une liaison articulée intermédiaire, caractérisé en ce que la liaison articulée intermédiaire présente deux liaisons pivotantes à l'élément articulé (9) situé côte flexion et deux liaisons pivotantes à l'élément articulé inférieur de raccordement (7), de telle sorte que lors d'un déplacement de la liaison pivotante, l'élément articulé (9) situé du côté flexion effectue par rapport à l'élément articulé inférieur de raccordement (7) un déplacement forcé qui comporte à la fois une translation et une rotation.

2. Liaison articulée selon la revendication 1, caractérisée en ce que l'élément articulé (9) situé du côté flexion est relié à l'élément articulé inférieur de raccordement (7) avec interposition d'au moins un élément d'accouplement (10, 11), la liaison de l'élément d'accouplement (10, 11) à la fois avec l'élément articulé (9) situé du côté flexion et avec l'élément articulé inférieur de raccordement (7) étant réalisée à rotation.

3. Liaison pivotante selon les revendications 1 ou 2, caractérisée en ce que l'élément articulé (9) situé du côté flexion est relié à l'élément articulé inférieur de raccordement (7) avec interposition de deux éléments d'accouplement (10, 11), la liaison des éléments d'accouplement (10, 11) à la fois avec l'élément articulé (9) situé du côté flexion et avec l'élément articulé inférieur de raccordement (7) étant réalisée à rotation, et les éléments (6, 7, 8, 9, 10, 11) forment une chaîne articulée forcée à six éléments selon Stephenson.

4. Liaison pivotante selon les revendications 1 ou 2, caractérisée en ce que l'élément articulé (9) situé du côté flexion est relié à l'élément articulé inférieur de raccordement (7) avec interposition d'au moins une chaîne articulée a trois éléments (20, 21), la chaîne articulée à trois éléments (20, 21) étant constituée d'un élément (22, 23) relié à rotation à l'élément articulé (9) situé du côté flexion, et qui de son côté est relié à rotation en deux endroits différents aux deux éléments de liaison (24, 25, 26, 27), qui de leur côté sont tous deux reliés à rotation à l'élément articulé inférieur de raccordement (7).

5. Liaison pivotante selon la revendication 4, caractérisée en ce que l'élément articulé (9) situé du côté flexion est relié à l'élément articulé inférieur de raccordement (7) avec interposition de deux chaînes articulées à trois éléments (20, 21).

6. Liaison pivotante selon l'une des revendications 1, 2 ou 4, caractérisée en ce que l'élément articulé (9) situé du côté flexion est relié à l'élément articulé inférieur de raccordement (7) au moyen d'au moins une articulation coulissante (15, 17; 16, 18) constituée de boulons et d'un guide de coulissement.

7. Liaison pivotante selon la revendication 6, caractérisée en ce que l'élément articulé (9) situé du côté flexion est relié à l'élément articulé inférieur de raccordement (7) au moyen de deux articulations à coulissement (15, 17; 16, 18) formées de boulons et de guides de coulissement.

8. Liaison pivotante selon l'une des revendications 1 à 7, caractérisée en ce qu'au moins un des éléments (6, 7, 8, 9, 10, 11) est prévu multiple.

9. Liaison pivotante selon l'une des revendications précédentes, caractérisée en ce qu'elle contient au moins un élément à ressort (12) qui est relié à au moins deux des éléments (6, 7, 8, 9, 10, 11).

10. Liaison pivotante selon la revendication 9, caractérisée en ce que l'élément à ressort (12) contient un amortisseur.
